(19) Europäisches Patentamt
European Patent Office
Office européen des brevets

(11) Numéro de publication: **0 095 431 B1**

(12) **FASCICULE DE BREVET EUROPEEN**

(45) Date de publication du fascicule du brevet: **02.10.85**

(51) Int. Cl.⁴: **C 07 C 69/44**, C 07 C 67/38, C 07 C 69/46, C 07 C 69/24

(21) Numéro de dépôt: **83420081.8**

(22) Date de dépôt: **05.05.83**

(54) **Procédé de préparation d'esters par carbonylation de composés monooléfiniques.**

(30) Priorité: **07.05.82 FR 8208120**

(43) Date de publication de la demande: **30.11.83 Bulletin 83/48**

(45) Mention de la délivrance du brevet: **02.10.85 Bulletin 85/40**

(84) Etats contractants désignés: **AT BE CH DE FR GB IT LI LU NL SE**

(56) Documents cités:
**US - A - 3 996 164**

**BULLETIN OF THE CHEMICAL SOCIETY OF JAPAN, vol. 46, no. 2, 1973, pages 524-530, Tokyo, JP. A. MATSUDA: *"The cobalt carbonyl-catalyzed hydroesterification of butadiene with carbon monoxide and methanol"***

(73) Titulaire: **RHONE-POULENC CHIMIE DE BASE, 25, quai Paul Doumer, F-92408 Courbevoie (FR)**

(72) Inventeur: **Jenck, Jean, 11, rue Lakanal, F-69100 Villeurbanne (FR)**

(74) Mandataire: **Varnière-Grange, Monique et al, RHONE-POULENC RECHERCHES Service Brevets Chimie et Polymères Centre de Recherches de Saint-Fons 85, avenue des Frères Perret B.P. 62, F-69192 St-Fons Cédex (FR)**

**EP 0 095 431 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet un procédé de préparation d'esters par carbonylation de composés monooléfiniques, c'est-à-dire par réaction du monoxyde de carbone et d'un alcool sur des composés renfermant une liaison oléfinique unique.

L'invention a plus spécifiquement pour objet, la préparation de diesters à partir de penténoates d'alkyles. Un objet particulier de la présente invention est la synthèse d'adipates d'alkyles par carbonylation des penténoates d'alkyles.

Il est bien connu, d'après le »Bulletin of the Chemical Society of Japan, vol. 46, 1973, pages 526 et 527«, qu'on obtient un mélange renfermant des diesters d'alkyle et notamment, un adipate d'alkyle, en faisant réagir du monoxyde de carbone et un alcool sur un pentène-3 oate d'alkyle, sous haute pression et à température élevée, en présence de cobalt carbonyle et d'une base azotée hétérocyclique et aromatique. Cependant le développement à l'échelle industrielle d'une telle technique, dont l'intérêt de principe n'est pas contesté, est largement compromis, non seulement par la faible efficacité du système catalytique, mais aussi par la proportion notable de pentanoate d'alkyle formée, bien que la réaction soit effectuée en l'absence d'hydrogène.

Le même type de problèmes est susceptible d'être rencontré dans le cas de la réaction du monoxyde de carbone et d'un alcool sur d'autres composés monooléfiniques, l'ampleur des problèmes variant toutefois avec la nature précise du substrat, comme le montre le brevet américain No. 3 996 164.

Il est par ailleurs bien connu des spécialistes de ce domaine que la présence de faibles quantités d'hydrogène dans le milieu réactionnel tend à augmenter l'efficacité des catalyseurs à base de cobalt, dans les procédés de synthèse d'esters par réaction d'un alcool et du monoxyde de carbone sur un composé oléfinique.

La Demanderesse a néanmoins constaté que dans la plupart des cas cet effet favorable, lié à la présence de faibles quantités d'hydrogène, est accompagné par une influence négative sur la sélectivité en esters linéaires, produits spécifiquement visés.

En effet, on observe que la présence d'hydrogène non seulement tend à augmenter la proportion de produits d'hydrogènation dans le mélange réactionnel, mais aussi elle est susceptible de diminuer la proportion d'ester linéaire parmi les esters formés.

Cet effet négatif grève lourdement l'économie de ces procédés dans la mesure où la valorisation des esters branchés et des produits d'hydrogénation est aléatoire, voire inexistante. Tel est notamment le cas des diesters branchés et des pentanoates d'alkyles produits lors de la carbonylation de penténoates d'alkyles. En effet ces produits étant en pratique détruits, leur formation correspond, en d'autres termes, à une perte intolérable de matière première. Par ailleurs de l'hydrogène peut être formé in-situ à partir des traces d'eau susceptibles d'être contenues par des réactifs de qualité technique, selon la réaction bien connue:

$$H_2O + CO \rightarrow CO_2 + H_2$$

Il serait souhaitable, pour des raisons économiques évidentes, de pouvoir utiliser du monoxyde de carbone de qualité technique renfermant de l'hydrogène, sans que cela se produise au détriment de la sélectivité en esters linéaires, esters visés. Il serait également souhaitable, pour les mêmes raisons, de pouvoir utiliser des réactifs renfermant des traces d'eau, sans que cela conduise à une perte de matière première.

Il a maintenant été trouvé de manière tout à fait surprenante qu'il est possible de préparer sélectivement des esters linéaires par réaction d'un alcool de formule et $R'-OH$ et du monoxyde de carbone sur un composé renfermant une liaison oléfinique unique de formule $R_1CH=CHR_2$, dans lesquelles:

— $R_1$ et $R_2$, identiques ou différents, représentent l'hydrogène ou un radical alkyle ayant au maximum 20 atomes de carbone, pouvant être substitué par 1 ou 2 atomes de chlore ou groupes alcoxy renfermant au maximum 4 atomes de carbone,

— $R_1$ pouvant en outre représenter un radical $-(CH_2)_p-COOH$, $-(CH_2)_p-COOR_3$ ou $-(CH_2)_p-CN$ dans lequel p est un entier au plus égal à 6 pouvant être nul, $R_3$ représente un radical alkyle comportant au maximum 12 atomes de carbone, un à deux groupements méthylène pouvant en outre comporter un substituant alkyle ayant au maximum 4 atomes de carbone,

$R_1$ et $R_2$ pouvant en outre former ensemble un radical unique bivalent $-(CH_2)_q-$, comportant le cas échéant un ou deux substituants alkyle ayant au maximum 4 atomes de carbone, q étant un entier compris entre 3 et 6 inclus, et R' est un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle, en présence de cobalt, d'une base azotée tertiaire à condition de conduire la réaction dans un hydrocarbure aromatique qui comporte de 1 à 3 substituants choisis parmi le groupement cyano et les radicaux de formule $R-Y-$ dans laquelle Y représente le lien valentiel, un atome d'oxygène, un atome de soufre, un groupe carbonyle ou un groupe carbonyloxy ($-CO-O-$), le radical R étant rattaché à

l'atome d'oxygène de (—CO—O—), R représente un radical alkyle, aralkyle ou aryle; ledit radical, qui peut comporter éventuellement un substituant cyano ou une entité divalente —O—; —CO— ou —CO—O— intercalée dans la chaîne carbonée principale, renferme au maximum 20 atomes de carbone; au moins un des substituants est choisi parmi le groupe cyano et les radicaux R—Y, dans lesquels Y est différent du lien de valence.

Selon le présent procédé on fait donc réagir du monoxyde de carbone et un alcool e formule R'—OH sur un composé de formule $R_1CH=CHR_2$, dans lesquelles R', $R_1$ et $R_2$ ont la signification donnée ci-avant.

Les matières de départ susceptibles d'être carbonylées selon le présent procédé son donc des composés renfermant une liaison oléfinique unique interne ou terminale; ces composés comportent plus spécifiquement de 3 à 20 atomes de carbone.

Par la mise en oeuvre du présent procédé on obtient des esters saturés, c'est-à-dire des composés qui renferment d'une part un groupement carboxylate (—COOR') et, d'autre part un atome d'hydrogène de plus que la matière de départ. Parmi ces esters prédomine le composé dont le groupement carboxylate (—COOR') est situé en position terminale sur la chaine principale de la matière de départ.

Une première catégorie de matières de dèpart plus particulièrement adaptées a pour formule:

$R_1CH=CHR_2$ dans laquelle $R_1$ et $R_2$, identiques ou différents représentent l'hydrogène ou un radical alkyle ayant au maximum 10 atomes de carbone, ou bien forment ensemble un radical unique bivalent $-(CH_2)_q-$ q ayant la signification précédente, ledit radical pouvant comporter le cas échéant 1 ou 2 substituants méthyle. A titre d'exemples de tels composés on peut citer le propylène, le butène-1, le butène-2, les hexènes, les octènes et le dodécène-1.

Une seconde catégorie de matières de départ plus particulièrement appropriées est constituée par les composés de formule:

$R_1CH=CHR_2$ dans laquelle $R_1$ représente un radical $-(CH_2)_p-COOR_3$, p et $R_3$ ayant la signification précédente, un à deux groupements méthylène pouvant comporter un substituant alkyle ayant au maximum 4 atomes de carbone, et $R_2$ représente l'hydrogène ou un radical alkyle ayant au maximum 4 atomes de carbone.

Parmi les composés de ce type les penténoates d'alkyles revênt un intérêt tout particulier, car ils permettent d'accéder aux adipates d'alkyles, intermédiaires de l'acide adipique.

Le présent procédé nécessite la mise en oeuvre d'un alcool de formule R'OH, R' ayant la signification donnée précédemment.

A titre d'exemples d'alcools utilisables dans le cadre du présent procédé on peut citer le méthanol, l'éthanol, l'isopropanol, le n-propanol, le tertio butanol, le n-hexanol, le cyclohexanol, l'éthyl-2 hexanol-1, le dodécanol-1, l'éthylène glycol, l'hexanediol-1,6, l'alcool benzylique, l'alcool phényléthylique et le phénol.

On utilise de préférence un alcanol ayant au maximum 4 atomes de carbone; le méthanol et l'éthanol conviennent bien à la mise en oeuvre du présent procédé.

On peut engager l'alcool et le composé monooléfinique en quantités stoechiométriques. Cependant on utilise de préférence un excès d'alcool, dans la proportion de 1 à 10, ou encore mieux, de 2 à 5 moles d'alcool par mole de composé monooléfinique.

La réaction est conduite en présence de cobalt. N'importe quelle source de cobalt susceptible de réagir avec le monoxyde de carbone dans le milieu réactionnel pour donner in-situ des complexes carbonyles du cobalt peut être utilisée dans le cadre du présent procédé.

Les sources typiques de cobalt sont par exemple le cobalt métallique finement divisé, des sels inorganiques tels que le nitrate ou le carbonate de cobalt, des sels organiques en particulier des carboxylates. Peuvent également être employés les cobalt-carbonyles ou hydrocarbonyles; le dicobaltoctacarbonyle convient bien à la mise en oeuvre du présent procédé.

Le rapport molaire entre le composé monooléfinique et le cobalt est en général compris entre 10 et 1000. Ce rapport est avantageusement fixé à une valeur comprise entre 20 et 300.

Le procédé selon la présente invention nécessite également la présence d'une base azotée tertiaire dont le $pK_a$ est compris entre 3 et 10.

La demanderesse préconise l'utilisation d'hétérocycles azotés formés de 5 à 6 chainons, pouvant comporter un ou deux substituants choisis parmi les groupes alkyles ou alcoxy ayant au maximum 4 atomes de carbone, le groupe hydroxy et les atomes d'halogéne, renfermant éventuellement 2 ou 3 doubles liaisons, et pouvant, par ailleurs, le cas échéant être soudés à un noyau benzénique, sous réserve que les maillons adjacents à l'hétéroatome d'azote ne soient ni substitués, ni communs à deux cycles.

Conviennent plus particulièrement à la mise en oeuvre du présent procédé les hétérocycles azotés à 6 chainons, de $pK_a$ compris entre 4 et 7, notamment la pyridine, la picoline-4, l'isoquinoléine et la lutidine-3,5.

La quantité de base azotée tertiaire utilisée est en général telle que le rapport molaire N/Co soit compris entre 1 et 50. Pour une bonne mise en oeuvre de l'invention la demanderesse préconise de fixer ce rapport à une valeur comprise entre 2 et 25.

L'une des caractéristiques essentielles du présent procédé réside dans l'utilisation à titre de solvant d'un hydrocarbure aromatique qui comporte de 1 à 3 substituants choisis parmi le groupement cyano

et les radicaux de formule R—Y— dans laquelle Y représente le lien valentiel, un atome d'oxygène, un atome de soufre, un groupe carbonyle ou un groupe carbonyloxy (—CO—O—), le radical R étant rattaché à l'atome d'oxygène de (—CO—O—), R représente un radical alkyle, aralkyle ou aryle; ledit radical qui peut comporter éventuellement un sustituant cyano ou une entité divalente —O—; —CO— ou —CO—O— intercalée dans la chaîne carbonée principale, renferme au maximum 20 atomes de carbone; au moins un des substituants est choisi parmi le groupe cyano et les radicaux R—Y, dans lesquels Y est différent du lien de valence.

Plus spécifiquement, on fait appel à des solvants pouvant être représentés par la formule:

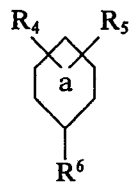

dans laquelle a est un noyau benzénique ou naphtalénique, $R_4$ représente un groupement cyano ou un radical R—Y, R et Y ayant la signification précédente, Y étant différent du lien valentiel, $R_5$ et $R_6$ identiques ou différents, représentent un atome d'hydrogène, un groupement cyano ou un radical R—Y, R et Y ayant la signification précédente, Y pouvant representer le lien valentiel. De préférence, l'un des radicaux $R_5$ et $R_6$ représente l'hydrogène ou un radical R—Y dans lequel Y est le lien valentiel et R est un radical alkyle ayant au maximum 4 atomes de carbone.

Lorsque Y représente un atome d'oxygène, un atome de soufre, ou un groupe carbonyle, R est plus particulièrement choisi parmi les radicaux alkyles, aralkyles ou aryles, comportant au maximum 10 atomes de carbone, et de préférence, R est un radical alkyle qui contient au maximum 4 atomes de carbone.

Lorsque Y représente un groupe carbonyloxy (—CO—O—) R est identique au radical R' de l'alcool utilisé comme matière de départ.

Lorsque Y représente le lien valentiel, R est plus particulièrement choisi parmi les radicaux alkyles ou aralkyles qui ont au maximum 10 atomes de carbone et qui peuvent comporter une entité divalente —O—, —CO— ou —CO—O— intercalée dans la chaîne carbonée, sous réserve de choisir R identique à R' lorsqu'une entité carbonyloxy est intercalée dans la chaîne; R peut aussi être un radical phényle éventuellement substitué par 1 à 3 radicaux alkyles ayant au maximum 4 atomes de carbone.

De préférence, a est un noyau benzénique, $R_4$ est avantageusement un radical R—Y— dans lequel Y représente un atome d'oxygène ou de soufre et R est un radical alkyle qui comporte au maximum 4 atomes de carbone ou un radical phényle. $R_5$ et $R_6$ representent de préférence un atome d'hydrogène.

Bien entendu, on peut utiliser des mélanges de plusieurs de ces composés aromatiques.

A titre d'exemples de solvants convenant à la mise en oeuvre du présent procédé on peut citer: le méthoxybenzène (anisole), le benzonirile, le méthylthiobenzène (thioanisole), l'éthoxybenzène (phénétole), l'oxyde de diphényle, le sulfure de diphényle, l'oxyde de phényle et de benzyle, le sulfure de phényle et de benzyle, l'acétophénone, la propiophénone, la n-butylphénylcétone, le benzoate de méthyle, le benzoate de n-butyle, le benzoate de phényle, le p-tolunirile, le méthoxy-2 toluène, l'éthoxy-2 toluène, la p-méthylacétophénone, le p-toluate de méthyle, le diméthoxy-1,2 benzène (vératrole), le diéthoxy-1,3 benzène, le diméthoxy-1,4 benzène, le téréphtalate de diméthyle, le p-méthoxybenzoate de méthyle, le p-éthylthiobenzoate de méthyle, la diméthyl-3,5 acétophénone et ses isomères, le triméthoxy-1,3,5 benzène . . .

L'anisole et le thioanisole conviennent particulièrement bien à la mise en oeuvre de la présente invention.

La quantité de solvant qui a une influence sur la sélectivité de la réaction sera en général supérieure à 20% (en poids) du mélange réactionnel initial et, pour une bonne mise en oeuvre du présent procédé, elle sera comprise entre 30 et 60% (en poids) dudit mélange.

Selon une variante avantageuse du présent procédé, la réaction est également conduite en présence d'hydrogène. Dans le cadre de cette variante, l'hydrogène représentera au moins 0,1% (en volume) du monoxyde de carbone, sans dépasser toutefois 3% (en volume). De préférence, la teneur en hydrogène représentera de 0,5 à 2% (en volume) du monoxyde de carbone.

Bien entenou, si l'hydrogène peut être introduit dans le milieu réactionnel, de manière commode, sous forme d'un mélange avec le monoxyde de carbone, il peut être aussi alimenté séparément.

La réaction est conduite en phase liquide à une température supérieure à 120° C, sans qu'il soit utile de dépasser 200° C, sous une pression de monoxyde de carbone d'au moins 50 bars et pouvant atteindre 1000 bars. On préfère opérer à une température de l'ordre de 130 à 180° C et sous une pression de monoxyde de carbone de l'ordre de 100 à 300 bars.

Bien entendu, les conditions de pression et de température optimales seront d'autant plus sévères que la matière de départ sera moins réactive, ce qui se produit, notamment, lorsque le degré de protection stérique de la double liaison augmente.

Le monoxyde de carbone utilisé peut renfermer, en plus de l'hydrogène, des impuretés telles que du

dioxyde de carbone, du méthane et de l'azote.

Comme cela a été indiqué en tête du présent mémoire, le procédé selon la présente invention trouve une application plus particulièrement intéressante dans la synthèse de diesters à partir de penténoates d'alkyles. En général, on met en oeuvre un pentène-3 oate d'alkyle, bien que les pentène-2 oates, les pentène-4 oates et des mélanges de penténoates d'alkyles puissent être utilisés. Dans le cadre de cette application, il s'avère préférable de choisir l'alcool (coréactif) corrspondant au reste alkyle de l'ester de départ, le reste alkyle ayant avantageusement 4 atomes de carbone du maximum. De bons résultats sont obtenus au départ de l'un ou l'autre des couples de réactifs suivants: penténoate de méthyle et méthanol, penténoate d'éthyle et éthanol.

En fin de réaction, ou lorsque la conversion souhaitée est atteinte on récupère l'ester linéaire recherché par tout moyen approprié, par exemple, par distillation ou extraction liquide-liquide.

Les exemples ci-après illustrent l'invention sans toutefois en limiter le domaine ou l'esprit.

Preparation d'adipate d'alkyle

Exemples 1 à 27

Essais témoins (a) à (i)

Les conventions suivantes sont utilisées ci-après.

Dans les produits formés ne sont pas inclus les composés résultant de l'isomérisation de position de la double liaison oléfinique.

Les produits formés sont essentiellement les diesters et le pentanoate d'alkyle, ce dernier résultant de l'hydrogénation de l'ester de départ.

— A désigne l'activité exprimée en moles de produits formés par heure et par atome-gramme de cobalt.
— X (%) désigne le nombre de moles de diesters pour 100 moles de produits formés.
— Y (%) désigne le nombre de moles d'adipate d'alkyle pour 100 moles de produits formés.
— Z (%) désigne le nombre de moles de pentanoate d'alkyle pour 100 moles de produits formés.

— Exemples 1 à 19 — Essais témoins (a) à (i):

On a réalisé une série d'essais selon le mode opératoire suivant:

Dans un autoclave de 125 ml en acier inoxydable, purgé sous argon on introduit du pentène-3 oate de méthyle (P3), du méthanol, du dicobaltoctacarbonyle (DCDC), de l'isoquinoléïne, et le cas échéant un solvant.

L'autoclave est alors purgé par un courant de monoxyde de carbone renfermant, le cas échéant, de l'hydrogène. On porte ensuite l'autoclave à la température (T), sous une pression (P). Après un temps de réaction (désigné par t et exprimé en heures), à cette température, l'autoclave est refroidi et dégazé. Le mélange réactionnel est analysé par chromatographie en phase gazeuse. Les conditions particulières ainsi que le résultats obtenus figurent respectivement dans les tableaux I(A) et I(B) ci-après:

Dans le tableau I(A) les rapports MeOH/P3, P3/Co et N/Co désignent respectivement le rapport molaire du méthanol au pentène-3 oate, le rapport du nombre de moles de pentène-3 oate au nombre d'atomes-grammes de cobalt, et le rapport du nombre de moles d'isoquinoléïne au nombre d'atomes-grammes de cobalt.

Dans le tableau I(B), DMMB signifie le diméthyl-3,5 méthoxybenzène et BM, le benzoate de méthyle.

Les essais témoins (a) (d) montrent clairement qu'en l'absence de solvant, la présence d'hydrogène se traduit par une augmentation de l'efficacité du catalyseur à base de cobalt et par une baisse notable de la sélectivité en adipate de diméthyle.

Les exemples 1 à 19 montrent que la présence simultanée d'un solvant selon l'invention et d'hydrogène permet d'obtenir sélectivement et efficacement l'adipate de diméthyle.

— Exemples 20 à 27:

Dans l'autoclave et selon le mode opératoire, décrits ci-avant on a réalisé une série d'essais sur une charge renfermant 50 mmol de pentène-3 oate de méthyle (exemples 20 à 22, 24 à 26) ou de pentène-2 oate de méthyle (P2, exemples 23 et 27), 100 mmol de méthanol, 1 mmol de dicobaltoctacarbonyle, 8 mmol d'isoquinoléïne et un solvant.

(P3 ou P2/Co = 25; MeOH/P3 ou P2 = 2; N/Co = 4)

Les conditions particulières ainsi que les résultats obtenus à 160° C sous 130 bars sont rassemblés dans le tableau II ci-après.

Tableau II

| Réf | $H_2$ (% vol) | t | Solvant nature | (% pds) | A | X (%) | Y (%) | Z (%) |
|---|---|---|---|---|---|---|---|---|
| 20 | 0,8 | 2 | anisole | 9 | 6,5 | 92,9 | 78,6 | 6,6 |
| 21 | 0,8 | 2 | anisole | 22 | 5,0 | 94,2 | 79,3 | 5,4 |
| 1 | 0,8 | 1,5 | anisole | 49 | 4,7 | 95,0 | 81,1 | 4,6 |
| 22 | 0,8 | 4 | anisole | 49 | 3,4 | 96,2 | 81,8 | 3,8 |
| 23 | 0,7 | 2 | anisole | 49 | 8,4 | 95,1 | 79,3 | 4,4 |
| 24 | 0,9 | 2 | thioanisole | 32 | 5,2 | 96,0 | 82,3 | 3,6 |
| 25 | 0,9 | 2 | thioanisole | 50 | 3,1 | 96,5 | 83,5 | 3,0 |
| 26 | 0,9 | 4 | thioanisole | 66 | 1,1 | 97,4 | 84,4 | 2,5 |
| 27 | 0,9 | 2 | thioanisole | 50 | 6,2 | 95,9 | 82,8 | 3,9 |

Preparation du pimelate de dimethyle

Exemple 28

Dans l'autoclave défini ci-avant et selon un mode opératoire analogue à celui décrit précédemment on réalise un essai sur une charge constituée de:

- 44 mmol d'hexène-2 oate de méthyle
- 100 mmol de méthanol
- 1,02 mmol de dicobaltoctacarbonyle
- 7,98 mmol d'isoquinoléïne
- 10,4 g de thioanisole (50% en poids)

En deux heures de réaction à 160° C, la pression totale en température étant maintenue constante et égale à 130 bars par des recharges périodiques de monoxyde de carbone renfermant 0,8% (en volume) d'hydrogène, on obtient les résultats suivants:

Le taux de transformation (TT) de l'hexène-2 oate de méthyle en produits de carbonylation et d'hydrogénation (à l'exclusion des produits d'isomérisation de la double liaison) est de: 34,3%.

La sélectivité (RT) des divers produits obtenus est respectivement:

- pimélate de diméthyle: 74%
- autres diesters en $C_7$: 18,5%
- hexanoate de méthyle: 7,5%

Preparation du nonandate de methyle

Exemple 29

essai témoin (j)

Dans l'autoclave défini ci-avant et selon un mode opératoire analogue à celui décrit précédemment, on réalise deux essais sur une charge comprenant:

- 50 mmol d'octène-2
- 100 mmol de méthanol
- 1 mmol de dicobaltoctacarbonyle
- 8 mmol d'isoquinoléïne

La charge de l'exemple 29 comprend en outre 10,4 g de thioanisole (50% en poids).

Les résultats obtenus respectivement dans l'exemple 29 et dans l'essai témoin (j), en deux heures de réaction à 160° C, sous une pression totale en température de 130 bars, maintenue constante par des recharges périodiques de monoxyde de carbone renfermant 0,8% (en volume d'hydrogène sont indiqués dans le tableau III ci-aprè, dans lequel RT et TT ont une signification analogue à celle donnée dans l'exemple 28

Tableau III

| Référence | 29 | j |
|---|---|---|
| TT (%) | 29,3 | 63 |
| RT (%) nonanoate de méthyle | 84,4 | 81,7 |
| RT (%) autres esters en $C_9$ | 15,1 | 18,0 |
| RT (%) octane | ≤0,5 | ≤0,3 |

Tableau I(A)

| Réf | P3 mmol | MeOH mmol | DCOC mol | MeOH/ P3 | P3/Co | N/Co | $H_2$ (% vol) | P bars | T °C |
|---|---|---|---|---|---|---|---|---|---|
| a | 50,1 | 109 | 1,02 | 2,17 | 24,6 | 4 | 0 | 130 | 160 |
| b | 49,7 | 99 | 0,88 | 1,99 | 28,5 | 4,5 | 0,7 | 130 | 160 |
| c | 99,8 | 198 | 2,00 | 1,98 | 25,0 | 12 | 0,9 | 130 | 160 |
| d | 99,8 | 200 | 2,01 | 2,00 | 24,8 | 12 | 2,6 | 130 | 160 |
| e | 50,3 | 104 | 1,00 | 2,07 | 25,2 | 3,9 | 0,8 | 130 | 160 |
| 1 | 50,8 | 102 | 1,00 | 2,01 | 25,4 | 4,0 | 0,8 | 130 | 160 |
| 2 | 50,2 | 102 | 1,01 | 2,03 | 24,8 | 4,0 | 0,8 | 130 | 160 |
| 3 | 50,1 | 101 | 0,96 | 2,01 | 26,0 | 4,3 | 0,8 | 130 | 160 |
| 4 | 49,3 | 99,4 | 1,04 | 2,02 | 23,6 | 4,0 | 0,8 | 130 | 160 |
| f | 101 | 196 | 1,97 | 1,94 | 25,6 | 8,1 | 0,8 | 130 | 160 |
| 5 | 49,8 | 100 | 0,92 | 2,00 | 27,0 | 8,6 | 0,8 | 130 | 160 |
| g | 49,7 | 98,4 | 1,01 | 1,98 | 24,5 | 7,9 | 0,8 | 130 | 180 |
| 6 | 50,3 | 103 | 0,88 | 2,05 | 28,6 | 9,1 | 0,8 | 130 | 180 |
| 7 | 50,0 | 98,0 | 0,92 | 1,96 | 27,0 | 8,5 | 0,8 | 130 | 180 |
| 8 | 50,4 | 101 | 1,04 | 2,01 | 24,0 | 7,8 | 0,8 | 130 | 180 |
| 9 | 50,5 | 99 | 1,00 | 1,96 | 25,3 | 8,0 | 0,8 | 130 | 180 |
| 10 | 49,8 | 100 | 1,01 | 2,01 | 24,7 | 4,1 | 0,8 | 130 | 180 |
| h | 50,1 | 98,8 | 1,00 | 1,97 | 25,1 | 4,0 | 0,8 | 130 | 180 |
| 11 | 50,3 | 102 | 1,03 | 2,03 | 25,2 | 4,0 | 0,8 | 130 | 140 |

Tableau I(A) (suit)

| Réf | P3 mmol | MeOH mmol | DCOC mol | MeOH/ P3 | P3/Co | N/Co | $H_2$ (% vol) | P bars | T °C |
|---|---|---|---|---|---|---|---|---|---|
| i | 49,7 | 100 | 1,03 | 2,01 | 24,1 | 3,8 | 0,8 | 130 | 140 |
| 12 | 100 | 202 | 2,07 | 2,02 | 24,2 | 4,0 | 0,8 | 130 | 160 |
| 13 | 50,1 | 100 | 1,02 | 2,00 | 24,6 | 4,0 | 0,8 | 130 | 160 |
| 14 | 50,0 | 100 | 0,94 | 2,00 | 26,5 | 4,1 | 0,9 | 130 | 137 |
| 15 | 50,4 | 100 | 1,01 | 1,98 | 24,9 | 3,8 | 0,9 | 130 | 152 |
| 16 | 50,4 | 101 | 1,02 | 2,00 | 24,7 | 8,0 | 0,9 | 130 | 180 |
| 17 | 100 | 1,99 | 1,00 | 1,99 | 50,2 | 8,0 | 0,8 | 250 | 180 |
| 18 | 50,4 | 103 | 0,99 | 2,04 | 25,4 | 3,9 | 2,0 | 130 | 160 |
| 19 | 50,5 | 101 | 0,99 | 2,00 | 25,4 | 12,1 | 0,9 | 130 | 160 |

Tableau I(B)

| Réf. | Solvant nature | (% pds) | t | A | X (%) | Y (%) | Z (%) |
|---|---|---|---|---|---|---|---|
| a | — | 0 | 1 | 3,7 | 95,1 | 79,4 | 4,9 |
| b | — | 0 | 1 | 10,4 | 89,0 | 74,6 | 10,4 |
| c | — | 0 | 1 | 1,9 | 94,7 | 75,5 | 4,9 |
| d | — | 0 | 1 | 3,3 | 92,5 | 74,7 | 6,8 |
| e | — | 0 | 2 | 7,9 | 92,3 | 76,4 | 7,0 |
| 1 | anisole | 49 | 1,5 | 4,7 | 95,0 | 81,1 | 4,6 |
| 2 | DMMB | 50 | 2 | 2,5 | 95,5 | 79,7 | 2,9 |
| 3 | vératrole | 51 | 2 | 5,1 | 94,4 | 79,4 | 5,1 |
| 4 | benzonitrile | 50 | 4 | 1,7 | 95,2 | 80,5 | 4,5 |
| f | — | 0 | 2 | 2,4 | 94,9 | 76,6 | 4,6 |
| 5 | anisole | 47 | 2 | 4,6 | 95,6 | 79,6 | 4,0 |
| g | — | 0 | 2 | 5,6 | 88,0 | 74,8 | 11,6 |
| 6 | anisole | 47 | 2 | 7,0 | 88,2 | 76,7 | 11,6 |
| 7 | acétophénone | 47 | 2 | 5,8 | 92,4 | 79,1 | 7,3 |
| 8 | benzonitrile | 46 | 2 | 1,0 | 91,7 | 81,2 | 8,3 |
| 9 | p-tolunitrile | 45 | 2 | 2,8 | 92,7 | 80,3 | 7,3 |
| 10 | anisole | 49 | 2 | 7,8 | 88,6 | 75,0 | 10,0 |

Tableau I(B) (suit)

| Réf. | Solvant nature | (% pds) | t | A | X (%) | Y (%) | Z (%) |
|---|---|---|---|---|---|---|---|
| h | — | 0 | 2 | 9,3 | 84,6 | 70,9 | 15,2 |
| 11 | anisole | 49 | 2 | 1,5 | 95,8 | 73,1 | 3,7 |
| i | — | 0 | 2 | 3,4 | 94,0 | 70,3 | 5,6 |
| 12 | BM | 35 | 2 | 3,7 | 95,3 | 79,4 | 4,1 |
| 13 | thioanisole | 49 | 1,5 | 2,9 | 96,5 | 83,9 | 3,2 |
| 14 | thioanisole | 33 | 2 | 0,9 | 96,2 | 76,1 | 2,8 |
| 15 | thioanisole | 50 | 2 | 2,9 | 96,4 | 83,2 | 3,0 |
| 16 | thioanisole | 50 | 2 | 5,0 | 92,4 | 79,6 | 7,6 |
| 17 | thioanisole | 34 | 2 | 5,6 | 96,6 | 82,4 | 3,2 |
| 18 | thioanisole | 50 | 2 | 4,4 | 93,7 | 81,1 | 5,2 |
| 19 | thioanisole | 45 | 2 | 1,9 | 96,0 | 80,8 | 3,6 |

**Revendications**

1. Procédé de préparation d'esters linéaires par réaction d'un alcool de formule R'—OH et du monoxyde de carbone sur un composé de formule $R_1CH=CHR_2$, dans lesquelles:

— $R_1$ et $R_2$, identiques ou différents, représentent l'hydrogène ou un radical alkyle ayant au maximum 20 atomes de carbone, pouvant être substitué par 1 ou 2 atomes de chlore ou groupes alcoxy renfermant au maximum 4 atomes de carbone,
— $R_1$ pouvant en outre représenter un radical $-(CH_2)_p-COOH$, $-(CH_2)_p-COOR_3$ ou $-(CH_2)_p-CN$ dans lequel p est un entier au plus égal à 6 pouvant être nul, $R_3$ représente un radical alkyle comportant au maximum 12 atomes de carbone, un à deux groupements méthylène pouvant en outre comporter un substituant alkyle ayant au maximum 4 atomes de carbone,

$R_1$ et $R_2$ pouvant en outre former ensemble un radical unique bivalent $-(CH_2)_q-$, comportant le cas échéant un ou deux substituants alkyle ayant au maximum 4 atomes de carbone, q étant un entier compris entre 3 et 6 inclus,

et R' est un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle, en présence de cobalt, d'une base azotée tertiaire et, le cas échéant d'hydrogène, caractérisé en ce que la réaction est conduite dans un hydrocarbure aromatique qui comporte de 1 à 3 substituants choisis parmi le groupement cyano et les radicaux de formule R—Y— dans laquelle Y représente le lien valentiel, un atome d'oxygène, un atome de soufre, un groupe carbonyle ou un groupe carbonyloxy (—CO—O—), le radical R étant rattaché à l'atome d'oxygène de (—CO—O—), R représente un radical alkyle, aralkyle ou aryle; ledit radical, qui peut comporter éventuellement un substituant cyano ou une entité divalente —O—, —CO— ou —CO—O— intercalée dans la chaîne carbonée principale, renferme au maximum 20 atomes de carbone; au moins un des substituants est choisi parmi le groupe cyano et les radicaux R—Y, dans lesquels Y est différent du lien valentiel.

2. Procédé selon la revendication 1, où dans le composé de formule $R_1CH=CHR_2$, $R_1$ et $R_2$, identiques ou différents représentent l'hydrogène ou un radical alkyle ayant au maximum 10 atomes de carbone, ou bien forment ensemble un radical unique bivalent $-(CH_2)_q-$ q ayant la signification donnée dans la revendication 1, ledit radical pouvant comporter le cas échéant 1 ou 2 substituants méthyle.

3. Procédé selon la revendication 1, où dans le composé de formule $R_1CH=CHR_2$ $R_1$ représente un radical $-(CCH_2)_p-COOR_3$, p et $R_3$ ayant la signification donnée dans la revendication 1, un à deux

groupements méthylène pouvant comporter un substituant alkyle ayant au mximum 4 atomes de carbone, et $R_2$ représente l'hydrogène ou un radical alkyle ayant au maximum 4 atomes de carbone.

4. Procédé selon la revendication 3, caractérisé en ce que le composé renfermant une liaison oléfinique unique est un penténoate d'alkyle.

5. Procédé selon l'une quelconque des revendications précédentes, caractérisé en ce que l'hydrocarbure a pour formule:

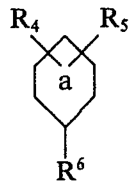

dans laquelle a est un noyau benzénique ou naphthalénique, $R_4$ représente un groupe cyano ou un radical R—Y, R et Y ayant la signification donnée dans la revendication 1, Y étant différent du lien valentiel, $R_5$ et $R_6$ identiques ou différents, représentent un atome d'hydrogène, un groupement cyano ou un radical R—Y, R et Y ayant la signification donnée dans la revendication 1, Y pouvant représenter le lien valentiel.

6. Procédé selon la revendication 5, caractérisé en ce que l'un des radicaux $R_5$ et $R_6$ représente l'hydrogène ou un radical R—Y dans lequel Y est le lien valentiel et R est un radical alkyle ayant au maximum 4 atomes de carbone.

7. Procédé selon la revendication 5 ou 6, caractérisé en ce que a est un noyau benzénique.

8. Procédé selon l'une quelconque des revendications 5 à 7, caractérisé en ce que $R_4$ est un radical R—Y— dans lequel Y représente un atome d'oxygène ou de soufre et R est un radical alkyle qui comporte au maximum 4 atomes de carbone.

9. Procédé selon l'une quelconque des revendications 5 à 8, caractérisé en ce que $R_5$ et $R_6$ représentent un atome d'hydrogène.

10. Procédé selon l'une quelconque des revendications précédentes, caractérisé en ce que l'hydrocarbure aromatique représente au moins 20% en poids du mélange réactionnel initial.

11. Procédé selon la revendication 10, caractérisé en ce que l'hydrocarbure aromatique représente de 30 à 60% en poids du mélange réactionnel initial.

12. Procédé selon l'une quelconque des revendications précédentes, caractérisé en ce que l'hydrogène représente au maximum 3% (en volume) du monoxyde de carbone.

13. Procédé selon l'une quelconque des revendications précédentes, caractérisé en ce que le rapport atomique N/Co est compris entre 1 et 50 et, de préférence entre 2 et 25.

14. Procédé selon l'une quelconque des revendications précédentes, caractérisé en ce que la température de réaction est comprise entre 120 et 200° C, de préférence, entre 130 et 180° C.

15. Procédé selon l'une quelconque des revendications précédentes, caractérisé en ce que la pression est comprise entre 50 et 1000 bars et, de préférence, entre 100 et 300 bars.

**Patentansprüche**

1. Verfahren zur Herstellung von linearen Estern durch Umsetzung eines Alkohols der Formel R'OH und von Kohlenmonoxid mit einer Verbindung der Formel $R_1CH=CHR_2$, in denen:

— $R_1$ und $R_2$ gleich oder verschieden sind und (jeweils) Wasserstoff oder eine Alkylgruppe mit maximal 20 Kohlenstoffatomen bedeuten, die durch 1 oder 2 Chloratome oder Alkoxygruppen mit maximal 4 Kohlenstoffatomen substituiert sein kann,

— $R_1$ auch für eine Gruppe $-(CH_2)_p-COOH$, $-(CH_2)_p-COOR_3$ oder $-(CH_2)_p-CN$ stehen kann, in der p eine ganze Zahl bis maximal 6 und auch 0 sein kann, $R_3$ eine Alkylgruppe mit maximal 12 Kohlenstoffatomen bedeutet und ein bis zwei Methylengruppen darüber hinaus einen Alkylsubstituenten mit maximal 4 Kohlenstoffatomen tragen können,

— $R_1$ und $R_2$ weiterhin zusammen eine einzige zweiwertige Gruppe $-(CH_2)_q-$ sein können, die gegebenenfalls ein oder zwei Alkylsubstituenten mit maximal 4 Kohlenstoffatomen aufweist, wobei q eine ganze Zahl von 3 bis 6 ist,

— und R' eine Alkylgruppe mit maximal 12 Kohlenstoffatomen, gegebenenfalls substituiert durch eine oder zwei Hydroxylgruppen, eine Cycloalkylgruppe mit 5 bis 7 Kohlenstoffatomen, eine Aralkylgruppe mit 7 bis 12 Kohlenstoffatomen oder eine Phenylgruppe ist, in Gegenwart von Cobalt, einer tertiären Stickstoffbase und gegebenenfalls Wasserstoff, dadurch gekennzeichnet, daß die Umsetzung in einem aromatischen Kohlenwasserstoff ausgeführt wird, der 1 bis 3 Substituenten trägt, die ausgewählt werden unter der Cyanogruppe und den Gruppen der Formel R—Y—, in der Y die (ko)valente Bindung, ein Sauerstoffatom, ein Schwefelatom, eine Carbonylgruppe oder eine Carbonyloxygruppe (—CO—O—) ist, wobei der Rest R an das Sauerstoffatom

von (—CO—O—) gebunden ist, R eine Alkyl-, Aralkyl- oder Arylgruppe bedeutet, diese Gruppe, die gegebenenfalls einen Cyanosubstituenten aufweist oder deren Kohlenstoffhauptkette durch eine zweiwertige Einheit —O—, —CO— oder —CO—O— unterbrochen ist, maximal 20 Kohlenstoffatome enthält, wobei zumindest einer der Substituenten ausgewählt ist unter der Cyanogruppe und den Gruppen R—Y, bei denen Y eine andere Bedeutung als die (ko)valente Bindung hat.

2. Verfahren nach Anspruch 1, bei dem in der Verbindung der Formel $R_1CH=CHR_2$ $R_1$ und $R_2$ gleich oder verschieden sind und (jeweils) für Wasserstoff oder eine Alkylgruppe mit maximal 10 Kohlenstoffatomen stehen oder zusammen eine einzige zweiwertige Gruppe $-(CH_2)_q$ bilden, wobei q die in Anspruch 1 angegebene Bedeutung hat und diese Gruppe gegebenenfalls 1 oder 2 Methylsubstituenten tragen kann.

3. Verfahren nach Anspruch 1, bei dem in der Verbindung der Formel $R_1CH=CHR_2$ $R_1$ eine Gruppe $-(CH_2)_p-COOR_3$ bedeutet, wobei p und $R_3$ die in Anspruch 1 angegebene Bedeutung haben, 1 bis 2 Methylengruppen einen Alkylsubstituenten mit maximal 4 Kohlenstoffatomen tragen können und $R_2$ Wasserstoff oder eine Alkylgruppe mit maximal 4 Kohlenstoffatomen ist.

4. Verfahren nach Anspruch 3, dadurch gekennzeichnet, daß die Verbindung, die eine einzige olefinische Bindung enthält, ein Alkylpentenoat ist.

5. Verfahren nach einem der vorangehenden Ansprüche, dadurch gekennzeichnet, daß der Kohlenwasserstoff der Formel

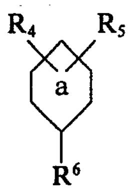

entspricht, in der a der Benzolring oder Naphthalinring ist, $R_4$ eine Cyanogruppe oder eine Gruppe R—Y ist, wobei R und Y die in Anspruch 1 angegebene Bedeutung haben und Y verschieden ist von der (ko)valenten Bindung, $R_5$ und $R_6$ gleich oder verschieden sind und (jeweils) für ein Wasserstoffatom, eine Cyanogruppe oder eine Gruppe R—Y stehen, wobei R und Y die im Anspruch 1 angegebene Bedeutung haben und Y die (ko)valente Bindung sein kann.

6. Verfahren nach Anspruch 5, dadurch gekennzeichnet, daß eine der Gruppen $R_5$ und $R_6$ Wasserstoff oder eine Gruppe R—Y ist, in der Y die (ko)valente Bindung und R eine Alkylgruppe mit maximal 4 Kohlenstoffatomen ist.

7. Verfahren nach Anspruch 5 oder 6, dadurch gekennzeichnet, daß a den Benzolring bezeichnet.

8. Verfahren nach einem der Ansprüche 5 bis 7, dadurch gekennzeichnet, daß $R_4$ eine Gruppe R—Y— ist, in der Y für ein Sauerstoff- oder Schwefelatom steht und R eine Alkylgruppe mit maximal 4 Kohlenstoffatomen ist.

9. Verfahren nach einem der Ansprüche 5 bis 8, dadurch gekennzeichnet, daß $R_5$ und $R_6$ (jeweils) für ein Wasserstoffatom stehen.

10. Verfahren nach einem der vorangehenden Ansprüche, dadurch gekennzeichnet, daß der aromatische Kohlenwasserstoff zumindest 20 Gew.-% des Ausgangs-Reaktionsgemisches ausmacht.

11. Verfahren nach Anspruch 10, dadurch gekennzeichnet, daß der aromatische Kohlenwasserstoff 30 bis 60 Gew.-% des Ausgangs-Reaktionsgemisches ausmacht.

12. Verfahren nach einem der vorangehenden Ansprüche, dadurch gekennzeichnet, daß der Wasserstoff maximal 3 Vol.-% des Kohlenmonoxids ausmacht.

13. Verfahren nach einem der vorangehenden Ansprüche, dadurch gekennzeichnet, daß das Atomverhältnis N/Co 1 bis 50, vorzugsweise 2 bis 25 beträgt.

14. Verfahren nach einem der vorangehenden Ansprüche, dadurch gekennzeichnet, daß die Reaktionstemperatur 120 bis 200° C, vorzugsweise 130 bis 180° C beträgt.

15. Verfahren nach einem der vorangehenden Ansprüche, dadurch gekennzeichnet, daß der Druck 50 bis 1000 bar, vorzugsweise 100 bis 300 bar ausmacht.

## Claims

1. Process for preparing linear esters by reacting an alcohol of formula R'OH and carbon monoxide with a compound of formula $R_1CH=CHR_2$, in which:

— $R_1$ and $R_2$, which may be identical or different, denote hydrogen or an alkyl radical which has a maximum of 20 carbon atoms and can be substituted with one or two chlorine atoms or alkoxy groups containing a maximum of four carbon atoms,

— $R_1$ being able, moreover, to denote a $-(CH_2)_p-COOH$, $-(CH_2)_p-COOR_3$ or $-(CH_2)_p-CN$ radical in which p is an integer at most equal to 6 and can be zero, $R_3$ denotes an alkyl radical

containing a maximum of 12 carbon atoms, one to two methylene groups being able, moreover, to contain an alkyl substituent having a maximum of 4 carbon atoms,

$R_1$ and $R_2$ being able, moreover, to form together a single bivalent radical $-(CH_2)_q-$ containing, where appropriate, one or two alkyl substituents having a maximum of 4 carbon atoms, q being an integer between 3 and 6 inclusive, and R' is an alkyl radical containing a maximum of 12 carbon atoms and optionally sustituted with one or two hydroxyl groups, a cycloalkyl radical having from 5 to 7 carbon atoms, and an aralkyl radical having from 7 to 12 carbon atoms or a phenyl radical, in the presence of cobalt, a tertiary nitrogenous base and, where appropriate, hydrogen, characterised in that the reaction is performed in an aromatic hydrocarbon which contains from 1 to 3 substituents chosen from the cyano group and radicals of formula $R-Y-$, in which Y denotes the valency link, an oxygen atom, a sulphur atom, a carbonyl group or a carbonyloxy ($-CO-O-$), group, the radical R being attached to the oxygen atom of ($-CO-O-$) and R denotes an alkyl, aralkyl or aryl radical; the said radical, which can optionally contain a cyano substituent or a divalent entity $-O-$, $-CO-$ or $-CO-O-$ inserted in the main carbon chain, contains a maximum of 20 carbon atoms; at least one of the substituents is chosen from the cyano group and $R-Y$ radicals in which Y is other than the valency link.

2. Process according to Claim 1, in which, in the compound of formula of $R_1CH=CHR_2$, $R_1$ and $R_2$, which may be identical or different, denote hydrogen or an alkyl radical having a maximum of 10 carbon atoms, or alternatively form together a single bivalent radical $-(CH_2)_q-$, q having the significance given in Claim 1 and the said radical can contain, where appropriate, 1 or 2 methyl substituents.

3. Process according to Claim 1, in which, in the compound of formula $R_1CH=CHR_2$, $R_1$ denotes a $-(CH_2)_p-COOR_3$ radical, p and $R_3$ having the significance given in Claim 1, one to two methylene groups can contain an alkyl substituent having a maximum of 4 carbon atoms, and $R_2$ denotes hydrogen or an alkyl radical having a maximum of 4 carbon atoms.

4. Process according to Claim 3, characterised in that the compound containing a single olefin bond is an alkyl pentenoate.

5. Process according to any one of the preceding claims characterised in that the hydrocarbon has the formula:

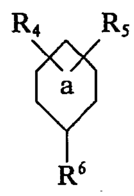

in which a is a benzene or naphthalene ring-system, $R_4$ denotes a cyano group or an $R-Y$ radical, R and Y having the significance given in Claim 1, Y being other than the valency link, $R_5$ and $R_6$, which may be identical or different, denote a hydrogen atom, a cyano group or an $R-Y$ radical, R and Y having the significance given in Claim 1, Y being able to denote the valency link.

6. Process according to Claim 5, characterised in that one of the radicals $R_5$ and $R_6$ denotes hydrogen or an $R-Y$ radical in which Y is the valency link and R is an alkyl radical having a maximum of 4 carbon atoms.

7. Process according to Claim 5 or 6, characterised in that a is a benzene ring.

8. Process according to any one of Claims 5 to 7, characterised in that $R_4$ is an $R-Y-$ radical in which Y denotes an oxygen or sulphur atom and R is an alkyl radical which contains a maximum of 4 carbon atoms.

9. Process according to any one of Claims 5 to 8, characterised in that $R_5$ and $R_6$ denote a hydrogen atom.

10. Process according to any one of the preceding claims, characterised in that the aromatic hydrocarbon represents at least 20% by weight of the initial reaction mixture.

11. Process according to Claim 10, characterised in that the aromatic hydrocarbon represents from 30 to 60% by weight of the initial reaction mixture.

12. Process according to any one of the preceding claims, characterised in that hydrogen represents a maximum of 3% (by volume) of the carbon monoxide.

13. Process according to any one of the preceding claims, characterised in that the atomic ratio N/Co is between 1 and 50 and preferably between 2 and 25.

14. Process according to any one of the preceding claims, characterised in that the reaction temperature is between 120 and 200°C, and preferably between 130 and 180°C.

15. Process according to any one of the preceding claims, characterised in that the pressure is between 50 and 1,000 bars, and preferably between 100 and 300 bars.